# FASCICULE DE BREVET EUROPEEN

(11) **EP 1 476 756 B1**
(45) Date de publication et mention de la délivrance du brevet: **02.09.2009**
(21) Numéro de dépôt: 03717420.8
(22) Date de dépôt: 20.02.2003
(51) Int. Cl.: G01N 33/552, B01L 3/00

(54) **COMPOSANT POUR MICROSYSTEME D'ANALYSE BIOLOGIQUE OU BIOCHIMIQUE**
KOMPONENTE FÜR EIN MIKROSYSTEM ZUR BIOLOGISCHEN ODER BIOCHEMISCHEN ANALYSE
COMPONENT FOR BIOLOGICAL OR BIOCHEMICAL ANALYSIS MICROFLUIDIC SYSTEM

(30) Priorité: 21.02.2002 FR 0202204
(43) Date de publication de la demande: 17.11.2004
(73) Titulaire: COMMISSARIAT A L'ENERGIE ATOMIQUE, 75015 Paris (FR)
(72) Inventeur: REVOL-CAVALIER, Frédéric, F-38180 SEYSSINS (FR); COMBETTE, Philippe, F-34170 CASTELNAU le LEZ (FR); TROUSSIER, Alain, F-38130 ECHIROLLES (FR)
(74) Mandataire: Poulin, Gérard
(86) Numéro de dépôt international: PCT/FR2003/000569
(87) Numéro de publication internationale: WO 2003/071278

(56) Documents cités:
- WO-A-01/04613
- WO-A-94/25862
- US-A- 5 008 358
- US-B1- 6 321 791
- "Flexible fused silica capillary tubing" POLYMICRO TECHNOLOGIES, [en ligne] octobre 1998 (1998-10), page 1 XP002228544 Extrait de l'Internet: <URL:www.optronis.com/docs/polymicro/tubep age.pdf> [extrait le 2003-01-24]
- DUFFY ET AL: "Rapid Prototyping of Microfluidic Systems in Poly(dimethylsiloxane)" ANALYTICAL CHEMISTRY, AMERICAN CHEMICAL SOCIETY. COLUMBUS, US, vol. 70, 1998, pages 4974-4984, XP002149044 ISSN: 0003-2700

## Description

### DOMAINE TECHNIQUE

La présente invention concerne un composant pour microsystème d'analyse biologique ou biochimique. Elle concerne également un procédé de réalisation d'un tel composant.

### ETAT DE LA TECHNIQUE ANTERIEURE

Un microsystème pour l'analyse biologique ou biochimique est réalisé à partir d'un support ou substrat choisi pour qu'une surface (qui peut être constituée de plusieurs zones) de ce support ou substrat apporte une ou plusieurs fonctions. Il peut s'agir d'une fonctionnalité chimique ou d'une fonctionnalité électrique.

La fonctionnalité chimique intervient lorsque des éléments biologiques ou biochimiques doivent être accrochés au support. Généralement, les supports sont en verre ou en silicium, ce qui permet l'accrochage des éléments biologiques ou biochimiques par une chimie de couplage bien maîtrisée, par exemple par silanisation.

La fonctionnalité électrique intervient pour la circulation de fluides dans des micro-canaux ou des micro-réservoirs. Les microsystèmes à circulation de fluides utilisent généralement des pompages électrocinétiques, tels que l'électro-osmose, pour faire circuler des fluides dans des micro-canaux et micro-réservoirs réalisés dans les supports. Ces modes de pompage imposent l'existence de surfaces électriquement actives. C'est l'utilisation de champs électriques élevés, combinés à la présence de surfaces électriquement actives, qui permet un écoulement fluidique. Les supports connus, en verre, en silice ou en silicium revêtu de silice, conviennent bien à ces modes de pompage.

Les supports en verre, en silice ou en silicium revêtu de silice conviennent donc bien à l'obtention des fonctionnalités chimique et électrique.

Il est de plus en plus fait référence à l'utilisation de matériaux inertes tels que les polymères, les plastiques, les colles, dans la réalisation de ces microsystèmes, voir par exemple Duffy et al., Anal Chem. 1998, 70, 1974-1984. Cependant, la chimie d'accrochage des éléments biologiques ou biochimiques sur ces matériaux inertes dépend de leur formulation chimique et reste délicate à mettre en oeuvre. Des matériaux tels que les plastiques moulés pour réaliser des micro-canaux, des polymères ou des résines photosensibles pour réaliser des microstructures seraient très largement utilisées dès lors qu'il serait possible d'y accrocher facilement des éléments biologiques ou biochimiques. En effet, ces matériaux sont de faible coût et sont utilisables en grande série.

Par ailleurs, les écoulements électrocinétiques sont problématiques dans des matériaux tels que les polymères classiques et nécessitent l'emploi de techniques lourdes telles que l'activation par plasma afin de générer des surfaces électriquement chargées. Cependant, il a été montré que ceci ne permet pas d'activer définitivement la surface traitée. Le système évolue donc dans le temps.

### EXPOSÉ DE L'INVENTION

La présente invention apporte une solution aux problèmes exposés ci-dessus. Elle permet l'utilisation de matériaux chimiquement inertes (les polymères, les résines, les plastiques, les colles, etc.) pour réaliser des supports de composants pour microsystèmes d'analyse biologique ou biochimique, les surfaces de ces matériaux étant traitées pour les rendre fonctionnalisables afin de permettre l'accrochage d'éléments biologiques ou biochimiques. Le traitement consiste à déposer sur les surfaces concernées une couche d'un matériau fonctionnalisable. Les éléments biologiques ou biochimiques peuvent ensuite être greffés par des techniques classiques, par exemple par une technique de silanisation.

Le matériau fonctionnalisable peut être déposé sur une surface plane du support ou sur une surface structurée du support qui peut être en plastique moulé, en polymère photosensible ou non ou une colle sérigraphiée. La couche de matériau fonctionnnalisable peut être déposée postérieurement à la réalisation d'un composant structuré. Elle peut également être gravée par les techniques utilisées en microtechnologie (gravure plasma ou chimique) afin de ne couvrir qu'une partie de la surface du composant.

Le matériau déposé peut aussi être un matériau procurant une fonctionnalité électrique au composant, ce qui autorise la circulation de fluides par pompage électrocinétique.

L'invention propose un composant pour microsystème d'analyse biologique ou biochimique formé à partir d'un support et possédant au moins une zone de surface fonctionnalisée chimiquement, pour y permettre la formation d'une chimie d'accrochage d'éléments biologiques ou biochimiques, et/ou électriquement, pour y permettre la formation de charges électriques, caractérisé en ce que le support comprend au moins une partie constituée d'un matériau inerte choisi parmi un polymère, un plastique, une résine et une colle, et recouverte d'une couche d'un matériau fonctionnalisé chimiquement et/ou électriquement pour fournir ladite zone de surface fonctionnalisée.

Le polymère peut être un polyimide.

Ladite partie peut former le support dans sa totalité.

Le support peut comprendre un substrat supportant ladite partie. Le substrat peut être en un matériau choisi parmi le verre, le silicium, un polymère et un métal.

Ladite partie peut être structurée.

Avantageusement, le matériau fonctionna-lisable est choisi parmi la silice, la silice synthétisée et le nitrure de silicium.

Ladite zone de surface peut supporter des fonctions chimiques aptes à assurer la fixation d'éléments biologiques ou d'autres fonctions chimiques sur ladite zone de surface.

Ladite zone de surface peut supporter des fonctions chimiques aptes à assurer la présence de charges électriques sur ladite zone de surface.

L'invention a pour objet un procédé de réalisation d'un composant pour microsystème d'analyse biologique ou biochimique à partir d'un support, le support devant présenter au moins une zone de surface fonctionnalisée chimiquement pour y permettre la formation d'une chimie d'accrochage d'éléments biologiques ou biochimiques, et/ou électriquement, pour y permettre la formation de charges électriques, caractérisé en ce qu'il comprend les étapes suivantes :
- préparation d'un support comprenant au moins une partie constituée d'un matériau inerte choisi parmi un polymère, un plastique, une résine et une colle,
- dépôt sur ladite partie d'une couche d'un matériau fonctionnalisable chimiquement et/ou électriquement choisi parmi la silice, la silice synthétisée et le nitrure de silicium pour fournir ladite zone de surface fonctionnalisée,
- fonctionnalisation chimique et/ou électrique du matériau fonctionnalisable.

L'étape de préparation peut comprendre la formation de ladite partie en matériau inerte sur un substrat. La partie en matériau inerte peut être formée par dépôt.

Eventuellement, avant l'étape de dépôt de la couche de matériau fonctionnalisable, ladite partie est soumise à une étape de structuration.

L'étape de préparation peut comprendre le moulage de ladite partie constituée d'un matériau inerte.

Le procédé peut comprendre en outre les étapes suivantes :
- avant l'étape de dépôt de la couche de matériau fonctionnalisable, le dépôt d'une couche de protection sur ladite partie à l'exclusion de la zone de surface devant être fonctionnalisée,
- après l'étape de dépôt de la couche de matériau fonctionnalisable sur la totalité de ladite partie, le lift-off de la couche de matériau fonctionnalisable recouvrant la couche de protection par élimination de la couche de protection.

Le dépôt de la couche de matériau fonctionnalisable peut être un dépôt de silice synthétisée obtenu par un procédé sol-gel.

Le dépôt de la couche de matériau fonctionnalisable peut être un dépôt de silice ou de nitrure de silicium obtenu par une technique d'évaporation ou de pulvérisation.

Le procédé peut comprendre en outre une étape de gravure de la couche de matériau fonctionnalisable. Cette gravure peut être une gravure plasma ou une gravure chimique.

### BRÈVE DESCRIPTION DES DESSINS

L'invention sera mieux comprise et d'autres avantages et particularités apparaîtront à la lecture de la description qui va suivre, donnée à titre d'exemple non limitatif, accompagnée des dessins annexés parmi lesquels :
- les figures 1A à 1C sont des vues en coupe illustrant la réalisation d'un premier composant pour microsystème d'analyse biologique ou biochimique selon l'invention,
- les figures 2A à 2C sont des vues en coupe illustrant la réalisation d'un deuxième composant pour microsystème d'analyse biologique ou biochimique selon l'invention.

### DESCRIPTION DÉTAILLÉE DE MODES DE RÉALISATION DE L'INVENTION

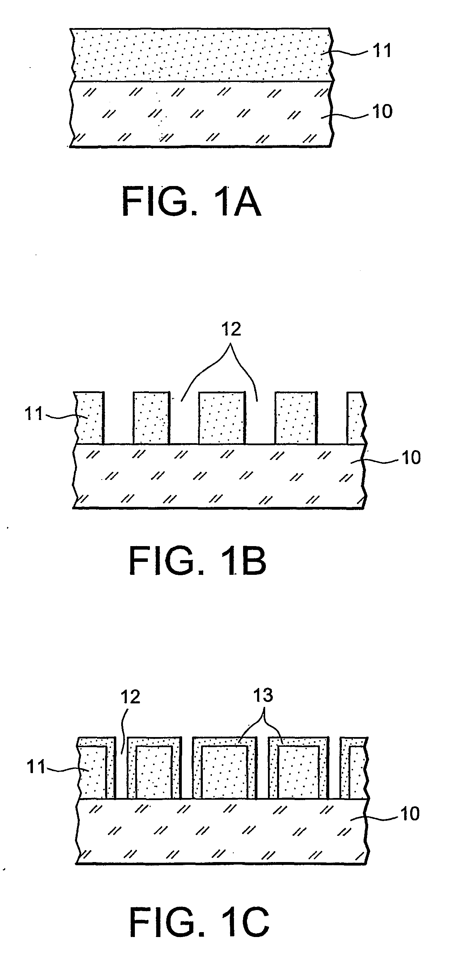

Les figures 1A à 1C illustrent la réalisation d'un composant pour microsystème d'analyse biologique ou biochimique à partir d'un substrat en verre.

La réalisation de microsystèmes biologiques ou biochimiques dans un substrat en verre est délicate. Au-delà de quelques micromètres de gravure, les impuretés contenues dans le verre bloquent la gravure. Le verre est donc gravé par voie chimique avec des solutions contenant de l'acide fluorhydrique. La gravure chimique du verre est une gravure isotrope, ce qui limite la forme et les dimensions des motifs réalisables. L'invention permet de s'affranchir de la gravure du verre en réalisant le composant dans un matériau structurable tel qu'un polymère photosensible ou non, une résine photosensible ou une colle. Ce matériau structurable est ensuite recouvert d'un matériau fonctionnalisable tel qu'une silice synthétique, une silice ou un nitrure de silicium obtenus par évaporation ou par pulvérisation, ou tout autre matériau fonctionnalisable que l'on peut déposer sur le matériau structurable.

La fonctionnalisation n'est plus réalisée directement sur le substrat en verre mais par l'intermédiaire de la couche de matériau fonctionnalisable déposée sur le matériau structurable. Le substrat supportant le matériau structurable peut alors être de nature diverse. Il peut être en verre, en silicium, en polymère, en métal ou en tout matériau sur lequel on peut déposer une couche de matériau structurable.

La figure 1A montre un substrat 10 constitué par une plaque de silicium de 100 mm de diamètre. Sur le substrat 10, une couche de matériau structurable 11 est déposée. Il s'agit d'un polymère polyimide photosensible commercialisé sous l'appellation "Probimide 7510". La couche 11 est déposée à la tournette, à une vitesse de 3000 tours/minute, puis recuite à 110°C sur une plaque chauffante. Le substrat 10 et la couche 11 forment un support.

La couche 11 est insolée par des rayons ultra-violets à travers un masque, puis développée afin d'obtenir le composant souhaité. C'est ce que montre la figure 1B. La couche 11 est structurée par la présence d'ouvertures 12 exposant le substrat 10.

Le polyimide est ensuite recuit à 150°C sur une plaque chauffante puis à 300°C dans un four de traitement thermique.

La figure 1C montre le composant obtenu après le dépôt d'une couche de matériau fonctionnalisable 13 sur la couche 11. Le matériau fonctionnalisable est par exemple de la silice synthétique commercialisée sous l'appellation "SOG 512" part la société Honeywell. La silice synthétique est déposée à la tournette à 1000 tours/minute puis recuite sur une plaque chauffante d'abord à 110°C puis à 250°C.

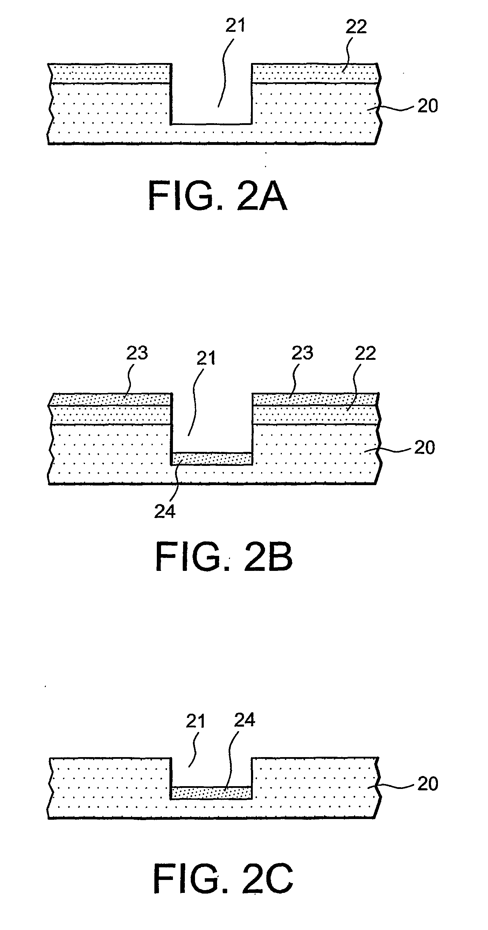

Les figures 2A à 2C illustrent la réalisation d'un composant à partir d'un plastique moulé présentant un mi-cro-canal fonctionnalisable.

L'utilisation du plastique moulé pour la réalisation de composants biologiques ou biochimiques est une technique très prometteuse dès lors que le composant peut être fonctionnalisé. L'invention permet la fonctionnalisation, par une silice synthétique, d'une partie de la structure moulée, par exemple un micro-canal.

La figure 2A montre un support 20 en plastique moulé, pourvu d'un canal 21 réalisé par le moulage. Sur la partie supérieure du support 20, une couche 22 de matériau de protection est déposée. Il peut s'agir d'une résine photosensible utilisée en microtechnologie. La résine est déposée à la tournette, à 2000 tours/minute puis recuite à 90°C à l'étuve.

Ensuite, comme le montre la figure 2B, une couche de silice, obtenue par évaporation d'une charge de silice dans un groupe d'évaporation, est déposée sur le support. La couche de silice forme un dépôt 23 sur la couche 22 de matériau de protection et un dépôt 24 sur le fond du canal 21.

La couche de protection est ensuite enlevée dans un bain chimique, par exemple dans un bain d'acétone. Sa dissolution entraîne le décrochage de la silice déposée à sa surface. Le résultat obtenu est visible sur la figure 2C. Seule la couche de silice 24 contenue au fond du canal 21 subsiste.

L'invention trouve une application dans tous les cas où l'on désire fonctionnaliser biologiquement ou biochimiquement une partie ou la totalité d'une surface d'un matériau inerte chimiquement à la fonctionnalisation. Elle trouve aussi une application lorsqu'il est nécessaire d'utiliser une couche de matériau électriquement actif.

Selon la nature du matériau fonctionnalisable, différentes techniques peuvent être employées pour le fonctionnaliser. Pour des matériaux tels que le silicium, l'oxyde de silicium, le nitrure de silicium ou la silice synthétique, un traitement de silanisation permet de fixer à la surface de ces matériaux des fonctions chimiques qui assureront ultérieurement la fixation d'éléments biologiques ou de fonctions chimiques.

Différents types de silanes peuvent être utilisés. Chacun possède son propre protocole de fixation à la surface du matériau à fonctionnaliser. Le choix du silane à employer dépend de la fonction chimique que l'on veut utiliser soit directement, soit pour la réalisation ultérieure d'une réaction chimique ou de la fixation d'un élément biologique. Parmi les silanes les plus couramment utilisés, on peut citer l'aminopropyltriéthoxysilane, l'aminopropyldiméthylé-thoxysilane, le silane époxy, le 2-(hydroxyéthyl)-3-aminopropyltriéthoxysilane.

A titre d'exemple, le protocole de silanisation utilisé pour l'aminopropyltriéthoxysilane est le suivant :
- traitement de la surface concernée par un plasma d'oxygène (Nextral 310) à 150 watts pendant 30 secondes pour créer sur la surface des fonctions silanol ;
- incubation dans une solution de silane à 10% dans de l'éthanol à 95% pendant 12 heures ;
- rinçage à l'eau distillée ;
- rinçage à l'éthanol à 95%
- recuit à 110°C pendant 3 heures en étuve.

On peut fixer directement des oligonucléotides synthétisés avec une fonction aldéhyde ou par l'intermédiaire d'un glutaraldéhyde si les oligonucléotides sont synthétisés avec une fonction NH₂.

Cette technique de silanisation permet de fixer des oligonucléotides, des protéines ou tout élément biologique ou chimique compatible avec les fonctions présentes sur le silane fixé au matériau fonctionnalisé (fonctions amine, acide aldéhyde, ester activé,...).

Si le matériau à fonctionnaliser est une couche d'or, on utilise la fixation de thiols ou de composés disulfurés sur la surface de cette couche métallique. Comme pour les silanes, différents thiols permettent d'obtenir à la surface de la couche à fonctionnaliser les fonctions chimiques nécessaires aux réactions chimiques voulues. Les techniques de fixation des thiols sur une surface métallique sont connues, par exemple par le document suivant : « Formation of Monolayer Films by the Spontaneous Assembly of Organic Thiols from Solution onto Gold » de C. D. BAIN et al., J. Am. Chem. Soc. 1989, Vol. 111, N° 1, pages 321 à 335.

Toujours à titre d'exemple, on peut citer le greffage d'acide mercapto-propionique ou de cystamine en incubant une solution à 1mM pendant 3 heures dans de l'éthanol absolu à température ambiante.

Pour une fonctionnalisation électrique, on peut obtenir des charges électriques en surface de silice synthétique, de silicium, de nitrure de silicium et d'oxyde de silicium en greffant un aminopropyltriéthoxysilane sur la couche à fonctionnaliser selon le protocole présenté ci-dessus. Un traitement en milieu acide (par exemple HCl 0,2M) permet de protéger le groupement amine du silane et d'obtenir des charges électriques en surface du matériau fonctionnalisé.

L'invention peut être avantageusement utilisée dans le domaine des systèmes d'analyse biologique et biochimique ainsi que dans les systèmes appelés "lab-on-chip".

## Revendications

1. Procédé de réalisation d'un composant pour microsystème d'analyse biologique ou biochimique à partir d'un support, le support devant présenter au moins une zone de surface fonctionnalisée chimiquement pour y permettre la formation d'une chimie d'accrochage d'éléments biologiques ou biochimiques, et/ou électriquement, pour y permettre la formation de charges électriques, **caractérisé en ce qu'**il comprend les étapes suivantes :
- préparation d'un support comprenant au moins une partie (11, 20) constituée d'un matériau inerte choisi parmi un polymère, un plastique, une résine et une colle,
- dépôt sur ladite partie (11, 20) d'une couche (13, 24) d'un matériau fonctionnalisable chimiquement et/ou électriquement choisi parmi la silice, la silice synthétisée et le nitrure de silicium pour fournir ladite zone de surface fonctionnalisée,
- fonctionnalisation chimique et/ou électronique du matériau fonctionnalisable.

2. Procédé selon la revendication 1,
**caractérisé en ce que** l'étape de préparation comprend la formation de ladite partie (11) en matériau inerte sur un substrat (10).

3. Procédé selon la revendication 2,
**caractérisé en ce que** ladite partie (11) en matériau inerte est formée par dépôt.

4. Procédé selon l'une quelconque des revendications 1 à 3, **caractérisé en ce que**, avant l'étape de dépôt de la couche de matériau fonctionnalisable, ladite partie (11) est soumise à une étape de structuration.

5. Procédé selon la revendication 1,
**caractérisé en ce que** l'étape de préparation comprend le moulage de ladite partie (20) constituée d'un matériau inerte.

6. Procédé selon l'une quelconque des revendications 1 à 5, **caractérisé en ce qu'**il comprend en outre les étapes suivantes :
- avant l'étape de dépôt de la couche de matériau fonctionnalisable, le dépôt d'une couche de protection (22) sur ladite partie (20) à l'exclusion de la zone de surface devant être fonctionnalisée,
- après l'étape de dépôt de la couche de matériau fonctionnalisable (23, 24) sur la totalité de ladite partie (20), le lift-off de la couche de matériau fonctionnalisable (23) recouvrant la couche de protection (22) par élimination de la couche de protection.

7. Procédé selon l'une quelconque des revendications 1 à 6, **caractérisé en ce que** le dépôt de la couche de matériau fonctionnalisable (13) est un dépôt de silice synthétisée obtenu par un procédé sol-gel.

8. Procédé selon l'une quelconque des revendications 1 à 6, **caractérisé en ce que** le dépôt de la couche de matériau fonctionnalisable est un dépôt (23, 24) de silice ou de nitrure de silicium obtenu par une technique d'évaporation ou de pulvérisation.

9. Procédé selon l'une quelconque des revendications 1 à 8, **caractérisé en ce qu'**il comprend en outre une étape de gravure de la couche de matériau fonctionnalisable.

10. Procédé selon la revendication 9,
**caractérisé en ce que** la gravure de la couche de matériau fonctionnalisable est une gravure plasma ou une gravure chimique.

## Claims

1. Method for producing a component for biological or biochemical analysis microsystems from a support, said support needing to have at least one chemically functionalised surface zone to allow in said zone the formation of a chemistry for anchoring biological or biochemical elements, and / or electrically, to allow in said zone the formation of electrical charges, **characterised in that** it comprises the following steps:
- preparing a support comprising at least one part (11, 20) formed of an inert material chosen from among a polymer, a plastics, a resin and an adhesive,
- depositing, on said part (11, 20), a layer (13, 24) of a chemically and / or electrically functionalisable material chosen from among silica, synthesised silica and silicon nitride in order to provide said functionalised surface zone,
- chemical and / or electrical functionalisation of the functionalisable material.

2. Method according to claim 1, **characterised in that** the preparation step comprises the formation of said part (11) in inert material on a substrate (10).

3. Method according to claim 2, **characterised in that** said part (11) in inert material is formed by deposition.

4. Method according to any of claims 1 to 3, **characterised in that**, before the step of depositing the layer of functionalisable material, said part (11) is subjected to a structuring step.

5. Method according to claim 1, **characterised in that** the preparation step comprises the moulding of said part (20) formed of an inert material.

6. Method according to any of claims 1 to 5, **characterised in that** it further comprises the following steps:
- before the step of depositing the layer of functionalisable material, depositing a protective layer (22) on said part (20) with the exception of the surface zone that is to be functionalised,
- after the step of depositing the layer of functionalisable material (23, 24) on the whole of said part (20), the lift-off of the layer of functionalisable material (23) covering the protective layer (22) by elimination of the protective layer.

7. Method according to any of claims 1 to 6, **characterised in that** the deposition of the layer of functionalisable material (13) is a deposition of synthesized silica obtained by a sol-gel method.

8. Method according to any of claims 1 to 6, **characterised in that** the deposition of the layer of functionalisable material is a deposition (23, 24) of silica or silicon nitride obtained by an evaporation or sputtering technique.

9. Method according to any of claims 1 to 8, **characterised in that** it further comprises a step of etching the layer of functionalisable material.

10. Method according to claim 9, **characterised in that** the etching of the layer of functionalisable material is a plasma etching or a chemical etching.

## Patentansprüche

1. Verfahren zur Herstellung einer Komponente für ein Mikrosystem zur biologischen oder biochemischen Analyse, basierend auf einem Träger, wobei dieser Träger wenigstens eine Oberflächenzone aufweist, die chemisch fiunktionalisieri ist, um dort die Bildung einer Chemie zur Befestigung biologischer oder biochemischer Elemente zu ermöglichen, oder elektrisch funktionalisiert ist, um dort die Bildung elektrischer Ladungen zu ermöglichen,
**dadurch gekennzeichnet, dass** es die folgenden Schritte umfasst:
- Präparierung eines Trägers mit wenigstens einem Teil (11, 20) aus inertem Material, ausgewählt unter einem Polymer, einem Kunststoff, einem Harz und einem Klebstoff,
- Abscheidung - auf dem genannten Teil (11,20) - einer Schicht (13, 24) aus einem chemisch und/oder elektrisch funktionalisierbaren Material, ausgewählt unter Siliciumdioxid, synthetisiertem Siliciumdioxid und Siliciumnitrid, um die genannte funktionalisierte Oberflächenzone zu realisieren,
- chemische und/oder elektronische Funktionalisierung des fiunktionalisierbaren Materials.

2. Verfahren nach Anspruch 1, **dadurch gekennzeichnet, dass** der Präparierungsschritt die Bildung des genannten Teils (11) aus inertem Material auf einem Substrat (10) umfasst.

3. Verfahren nach Anspruch 2, **dadurch gekennzeichnet, dass** der genannte Teil (11) aus inertem Material durch Abscheidung gebildet wird,

4. Verfahren nach einem der Ansprüche 1 bis 3, **dadurch gekennzeichnet, dass** der genannte Teil (11) vor dem Abscheidungsschritt der Schicht aus funktionalisierbarem Material einem Strukturierungsschritt unterzogen wird.

5. Verfahren nach Anspruch 1, **dadurch gekennzeichnet, dass** der Präparierungsschritt das Gießen des genannten, durch ein inertes Material gebildeten Teiles (20) umfasst.

6. Verfahren nach einem der Ansprüche 1 bis 5, **dadurch gekennzeichnet, dass** es außerdem die folgenden Schritte umfasst:
- vor dem Abscheidungsschritt der Schicht aus funktionalisierbarem Material
- das Abscheiden einer Schutzschicht (22) auf dem genannten Teil (20) mit Ausnahme der Oberflächenzone, die funktionalisiert werden soll,
- nach dem Abscheidungsschritt der Schicht aus funktionaüsierbarem Material (23, 24) auf dem gesamten genannten Teil (20) - das Lift-off der die Schutzschicht (22) bedeckenden Schicht aus fiunktionalisierbarem Material (23) durch Eliminierung der Schutzschicht.

7. Verfahren nach einem der Ansprüche 1 bis 6, **dadurch gekennzeichnet, dass** die Abscheidung der Schicht aus funktionalisierbarem Material (13) eine Abscheidung aus synthetisiertem Siliciumdioxid ist, hergestellt durch ein Sol-Gel-Verfahren.

8. Verfahren nach einem der Ansprüche 1 bis 6, **dadurch gekennzeichnet, dass** die Abscheidung der Schicht aus funktionalisierbarem Material eine Abscheidung (23, 24) aus Siliciumdioxid oder Siliciumnitrid ist, hergestellt durch eine Verdampfungs- oder Zerstäubungstechnik.

9. Verfahren nach einem der Ansprüche 1 bis 8, **dadurch gekennzeichnet, dass** es außerdem einen Schritt zur Ätzung der Schicht aus funktionalisierbarem Material umfasst.

10. Verfahren nach Anspruch 9, **dadurch gekennzeichnet, dass** die Ätzung der Schicht aus funktionalisierbarem Material eine Plasmaätzung oder eine chemische Ätzung ist.
